# EUROPEAN PATENT APPLICATION

(11) **EP 4 450 574 A1**
(43) Date of publication of application: **23.10.2024**
(21) Application number: 22932301.9
(22) Date of filing: 10.11.2022
(51) Int. Cl.: C09D 17/00, A61Q 1/00, A61Q 17/04, A61K 8/19, A61K 8/29, C09C 3/08

(54) **PIGMENT COMPOSITION, COSMETIC MATERIAL, INK, COATING MATERIAL, TONER, AND MOLDED ARTICLE**

(30) Priority: 15.03.2022 JP 2022040060
(71) Applicant: DIC Corporation, Tokyo 174-8520 (JP)
(72) Inventor: SUGIURA Kenichi, Kamisu-shi, Ibaraki 314-0193 (JP); MIYOSHI Ayaka, Kamisu-shi, Ibaraki 314-0193 (JP); KIMURA Akira, Kamisu-shi, Ibaraki 314-0193 (JP); MORIMITSU Taro, Kamisu-shi, Ibaraki 314-0193 (JP)
(74) Representative: TBK
(86) International application number: PCT/JP2022/041829
(87) International publication number: WO 2023/176038

(57) **Abstract**

An object to be achieved by the present invention is to provide a pigment composition having satisfactory slipperiness, for example, when used in cosmetic materials and having a glossy appearance, and a cosmetic material, an ink, a coating material, a toner, or a molded article, which contains the pigment composition.

The pigment composition of the present invention is an inorganic pigment coated with lauroyl lysine, in which a contact angle with respect to water is 95 degrees or greater, and a nitrogen specific surface area ratio between before and after coating is 0.70 or less. Further, the pigment composition of the present invention is an organic pigment coated with lauroyl lysine, in which a contact angle with respect to water is 95 degrees or greater, and a nitrogen specific surface area ratio between before and after coating is 0.90 or less. The cosmetic material, the ink, the coating material, the toner, or the molded article of the present invention contains the pigment composition described above.

## Description

### Technical Field

The present invention relates to a pigment composition, and a cosmetic material, an ink, a coating material, a toner, or a molded article, which contains the pigment composition.

### Background Art

Base makeup cosmetic materials such as sunscreen, makeup foundation, and foundation and point makeup cosmetic materials such as lipsticks, eyeliners, blusher, and nail polish are required to have spreadability when applied to the skin in order to enhance a sense of finish obtained when applied to the skin. However, since inorganic pigments such as titanium oxide fine particles or organic pigments obtained by making dyes insoluble in water are used as pigment components of the cosmetic materials, and the surfaces of the particles are hydrophilic, the dispersibility in the cosmetic materials which are in an oil state is low, the spreadability when the cosmetic materials are applied to the skin is degraded, and thus color unevenness may be caused. Therefore, the pigments used in the cosmetic materials are required to be subjected to a surface treatment such that the pigments have combined performance such as satisfactory compatibility with oil, satisfactory slipperiness when the cosmetic materials are applied to the skin, and a satisfactory appearance such as gloss.

Pigments having a surface coated with a functional substance are known as the pigments used in cosmetic materials. For example, a cosmetic material that contains an inorganic pigment subjected to a surface treatment with a N-mono-long-chain acyl basic amino acid containing one aliphatic acyl group having 8 to 22 carbon atoms in a molecule is known as such a coated pigment (PTL 1). Further, a surface modifier for an inorganic filler, obtained by coating a surface of an inorganic pigment with specific N-ε-acyl lysine, is known (PTL 2).

### Citation List

### Patent Literature

PTL 1: Japanese Unexamined Patent Application Publication No. 61-214257
PTL 2: Japanese Unexamined Patent Application Publication No. 60-67565

### Summary of Invention

### Technical Problem

It has been found that the coated pigment and the modifier described in PTLs 1 and 2 have large surface unevenness and poor slipperiness, for example, when used in cosmetic materials, and thus have a poor appearance such as gloss (see Comparative Example 1 of the present application). Further, PTL 2 only describes, as the effect of the surface modifier for an inorganic filler obtained by being coated with N-acyl lysine, the dispersion state of the modifier in an organic solvent, and does not clearly describes the effects thereof on the degree of hydrophobicity and the hue, and the touch feeling. Therefore, there is a demand for a coated pigment having satisfactory slipperiness, for example, when used in cosmetic materials and having a glossy appearance.

An object of the present invention is to solve the above-described problems of the coated pigment and to provide a pigment composition having satisfactory slipperiness, for example, when used in cosmetic materials and having a glossy appearance, and a cosmetic material, an ink, a coating material, a toner, or a molded article, which contains the pigment composition.

### Solution to Problem

As a result of intensive examination conducted by the present inventors, it has been found that the above-described problems can be solved by setting the contact angle of the surface of an inorganic or organic pigment coated with lauroyl lysine to a certain value or greater and setting the nitrogen specific surface area ratio between before and after coating to a certain value or greater, thereby completing the present invention.

That is, the present invention relates to
"Item 1. A pigment composition which is an inorganic pigment coated with lauroyl lysine, in which a contact angle with respect to water is 95 degrees or greater, and a nitrogen specific surface area ratio between before and after coating is 0.70 or less.
Item 2. A pigment composition which is an organic pigment coated with lauroyl lysine, in which a contact angle with respect to water is 95 degrees or greater, and a nitrogen specific surface area ratio between before and after coating is 0.90 or less.
Item 3. The pigment composition according to Item 1, in which the inorganic pigment is a metal and/or a metal oxide.
Item 4. The pigment composition according to Item 3, in which the metal oxide is at least one or more selected from the group consisting of titanium oxide and iron oxide.
Item 5. The pigment composition according to Item 2, in which the organic pigment is at least one or more selected from Blue No. 1, Red No. 202, and Yellow No. 4.
Item 6. A cosmetic material, an ink, a coating material, a toner, or a molded article, which contains the pigment composition according to any one of Items 1 to 5." Advantageous Effects of Invention

The pigment composition of the present invention has a satisfactory touch feeling and is smooth in a coated material, and thus the gloss increases. Therefore, the pigment composition is suitable for applications in cosmetic materials, particularly base makeup cosmetic materials such as sunscreen, makeup foundation, and foundation and point makeup cosmetic materials such as lipsticks, eyeliners, blusher, and nail polish.

### Description of Embodiments

Hereinafter, the present invention will be described in detail.

### [Pigment composition]

A pigment composition of the present invention is an inorganic or organic pigment obtained by coating the surface of particles with lauroyl lysine, and the contact angle of the surface with respect to water is 95 degrees or greater. Further, the nitrogen specific surface area ratio between before and after coating of the inorganic pigment with lauroyl lysine is 0.70 or less, and the nitrogen specific surface area ratio between before and after coating of the organic pigment with lauroyl lysine is 0.90 or less. In the present invention, the concept "coating" includes not only a case where the surface of particles is completely coated, but also a case where only a part of the surface is coated (for example, "adhesion"). Further, the expression "nitrogen specific surface area ratio between before and after coating" denotes the nitrogen specific surface area ratio of the pigment after the coating treatment to the pigment before the coating treatment with lauroyl lysine (after coating treatment/before coating treatment; BET (nitrogen) untreated ratio).

In a case where the pigment composition of the present invention is an inorganic pigment, the contact angle with respect to water is preferably 105 degrees or greater, more preferably 110 degrees or greater, and still more preferably 115 degrees or greater. Further, in a case where the pigment composition is an organic pigment, the contact angle with respect to water is preferably 95 degrees or greater and more preferably 100 degrees or greater. When the contact angle is a certain value or greater as described above, the touch feeling in a coated material is satisfactory and the surface thereof is smooth, and thus gloss increases. The pigment composition of the present invention exhibits the effects when the contact angle is 95 degrees or greater regardless of whether the pigment composition is an inorganic pigment or an organic pigment, but the inorganic pigment is required to have a higher contact angle and a lower nitrogen specific surface area ratio between before and after coating as compared with those of the organic pigment. The reason for this is considered to be that an inorganic pigment typically has a hydrophilicity higher than that of an organic pigment, and thus the surface of the inorganic pigment is required to be modified with lauroyl lysine more than that of the organic pigment in order to improve the dispersibility in oils such as cosmetic materials to exhibit the performance. The contact angle with respect to water can be determined by dropping 1.0 µL of water on a pigment tablet and measuring the contact angle 1.0 seconds after the dropping with a contact angle meter (liquid droplet method).

In a case where the pigment composition of the present invention is an inorganic pigment, the nitrogen specific surface area ratio between before and after coating is preferably 0.75 or less and more preferably 0.70 or less. Further, in a case where the pigment composition is an organic pigment, the nitrogen specific surface area ratio between before and after coating is preferably 0.95 or less and more preferably 0.90 or less. When the nitrogen specific surface area ratio is a certain value or less as described above, the touch feeling in the coated material is satisfactory and the surface thereof is smooth, and thus the gloss increases. The nitrogen specific surface area ratio can be determined by determining the nitrogen specific surface area (gas adsorption BET) between before and after the coating treatment using a gas adsorption measuring device and calculating the nitrogen specific surface area ratio of the pigment after the coating treatment to the pigment before the coating treatment (BET (nitrogen) untreated ratio).

The water vapor/nitrogen specific surface area ratio of the pigment composition according to the present invention by a gas adsorption method is, for example, in a range of 0.1 to 0.9, preferably in a range of 0.4 to 0.9, and more preferably in a range of 0.6 to 0.8. When the specific surface area ratio is in the above-described ranges, the touch feeling in the coated material is satisfactory and surface thereof is smooth, and thus gloss increases. The water vapor/nitrogen specific surface area ratio can also be determined by determining the specific surface areas (gas adsorption BET) of water vapor and nitrogen using a gas adsorption measuring device and calculating the ratio of the water vapor specific surface area to the nitrogen specific surface area. Further, the water vapor specific surface area is, for example, in a range of 1 to 300 m²/g and preferably in a range of 1 to 150 m²/g, and the nitrogen specific surface area is, for example, in a range of 1 to 300 m²/g and preferably in a range of 1 to 150 m²/g.

The primary particle diameter of the pigment composition according to the present invention is preferably 1.0 um or less, more preferably in a range of 0.001 to 0.8 um, and still more preferably in a range of 0.005 to 0.6 um. In a case where the primary particle diameter thereof is in the above-described ranges, the touch feeling in the coated material is satisfactory and the surface thereof is smooth, and thus the gloss is satisfactory. Further, in a case where the pigment composition of the present invention is aggregated to form secondary particles, the particle diameter thereof is preferably in a range of 0.005 to 2.0 um and more preferably in a range of 0.01 to 1.0 µm.

### (Inorganic pigment)

It is preferable that the inorganic pigment of the pigment composition according to the present invention be a metal and/or a metal oxide. Examples of the metal include silicon (Si), iron (Fe), aluminum (Al), sodium (Na), calcium (Ca), magnesium (Mg), potassium (K), copper (Cu), manganese (Mn), titanium (Ti), silver (Ag), gold (Au), platinum (Pt), lead (Pb), chromium (Cr), tin (Sn), molybdenum (Mo), gallium (Ga), and indium (In). Further, examples of the metal oxide include oxides of the metals described above, such as titanium oxide, iron oxide, yellow iron oxide, black iron oxide, zinc oxide, chromium oxide, zirconium oxide, magnesium oxide, and alumina. In the pigment composition of the present invention, it is preferable that the metal or the metal oxide be at least one or more selected from the group consisting of titanium oxide and iron oxide. In a case where the metal or the metal oxide is titanium oxide or iron oxide, the effect that the touch feeling in the coated material is satisfactory and the surface thereof is smooth, and thus the gloss increases can be further exhibited. These metals or metal oxides may be used alone or in combination of a plurality of kinds thereof.

### (Organic pigment)

Examples of the organic pigment in the pigment composition according to the present invention include azo-based, phthalocyanine-based, anthraquinone-based, perylene-based, perinone-based, quinacridone-based, thioindigo-based, dioxazine-based, isoindolinone-based, quinophthalone-based, azomethine-based, diketopyrrolopyrrole-based, and isoindoline-based pigments, and examples thereof include Red No. 3, Red No. 104, Red No. 106, Red No. 201, Red No. 202, Red No. 204, Red No. 205, Red No. 220, Red No. 223, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 401, Blue No. 1, Blue No. 404, C.I. Pigment Red 1, 2, 3, 4, 5, 6, 7, 8, 9, 12, 14, 15, 16, 17, 21, 22, 23, 31, 32, 37, 38, 41, 47, 48, 48:1, 48:2, 48:3, 48:4, 49, 49:1, 49:2, 50:1, 52:1, 52:2, 53, 53:1, 53:2, 53:3, 57, 57:1, 57:2, 58:4 , 60, 63, 63:1, 63:2, 64, 64:1, 68, 69, 81, 81:1, 81:2, 81:3, 81:4, 83, 88, 90:1, 101, 101:1, 104, 108, 108:1, 109, 112, 113, 114 , 122, 123, 144, 146, 147, 149, 151, 166, 168, 169, 170, 172, 173, 174, 175, 176, 177, 178, 179, 181, 184, 185, 187, 188, 190, 193, 194, 200, 202, 206, 207, 208, 209, 210, 214, 216, 220, 221, 224, 230, 231, 232, 233, 235, 236, 237, 238, 239, 242, 243, 245, 247, 249, 250, 251, 253, 254, 255, 256, 257, 258, 259, 260, 262, 263, 264, 265, 266, 267, 268, 269, 270, 271, 272, 273, 274, 275, 276, 277, 278, 279, 280, 281, 282, 283, 284 , 285, 286, 287, 291, 295, and 296, C.I. Pigment Blue 1, 1:2, 9, 14, 15, 15:1, 15:2, 15:3, 15:4, 15:6, 16, 17, 19, 25, 27, 28, 29, 33, 35, 36, 56, 56:1, 60, 61, 61:1, 62, 63, 66, 67, 68, 71, 72, 73, 74, 75, 76, 78, and 79, C.I. Pigment Yellow 1, 2, 3, 4, 5, 10, 12, 13, 14, 15, 16, 17, 18, 24, 31, 32, 34, 35, 35:1, 36, 36:1, 37, 37:1, 40, 42, 43, 53, 55, 60, 61, 62, 63, 65, 73, 74, 77, 81, 83, 93, 94, 95, 97, 98, 100, 101, 104, 106, 108, 109, 110, 113, 114, 115, 116, 117, 118, 119, 120, 123, 126, 127, 128, 129, 139, 147, 150, 151, 152, 153, 154, 155, 156, 161, 162, 164, 166, 167, 168, 169, 170, 171, 172, 173, 174, 175, 176, 177, 179, 180, 181, 182, 185, 187, 188, 192, 193, 194, 196, 198, 199, 213, and 214, C.I. Pigment Violet 1, 1:1, 2, 2:2, 3, 3:1, 3:3, 5, 5:1, 14, 15, 16, 19, 23, 25, 27, 29, 31, 32, 37, 39, 42, 44, 47, 49, and 50, and C.I. Pigment Green 1, 2, 4, 7, 8, 10, 13, 14, 15, 17, 18, 19, 26, 36, 37, 45, 48, 50, 51, 54, 55, 58, and 59. Among these, at least one or more selected from Brilliant Blue FCF (Blue No. 1), Lithol Rubine BCA (Red No. 202), and Tartrazine (Yellow No. 4) are preferable as the organic pigment. These organic pigments may be used alone or in combination of a plurality of kinds thereof. Further, the inorganic or organic pigment in the present invention may be a composite combination of the above-described metals or metal oxides and the organic pigments.

As the size of the particles of the inorganic or organic pigment, the diameter thereof is, for example, in a range of 0.001 to 1.0 um and preferably in a range of 0.005 to 0.6 µm.

The content of the inorganic or organic pigment is, for example, in a range of 15% to 98% by mass and preferably in a range of 30% to 95% by mass with respect to the total amount of the pigment composition. In a case where the proportion of the inorganic or organic pigment is in the above-described ranges, the touch feeling in the coated material is satisfactory and the surface thereof is smooth, and thus the gloss is satisfactory.

The pigment composition of the present invention is an inorganic or organic pigment coated with lauroyl lysine, but an amino acid may be used together with lauroyl lysine. N-acyl amino acid is preferable as such an amino acid.

Examples of the N-acyl amino acid include an acyl N-methyl amino acid salt such as sodium methyl cocoyl taurate, potassium methyl cocoyl taurate, sodium methyl lauroyl taurate, sodium methyl myristoyl taurate, sodium lauroyl methyl alanine, sodium lauroyl sarcosinate, triethanolamine lauroyl sarcosinate, or sodium methyl alanine lauroyl glutamate; sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alanine. These N-acyl amino acids may be used alone or in combination of a plurality of kinds thereof.

The content of the lauroyl lysine in the pigment composition according to the present invention is, for example, in a range of 0.5 to 10 parts by mass and preferably in a range of 1 to 5 parts by mass with respect to 100 parts by mass of the inorganic or organic pigment. Further, the content of the lauroyl lysine is, for example, in a range of 0.5% to 8% by mass and preferably in a range of 1% to 5% by mass with respect to the total amount of the pigment composition. In a case where the proportion of the lauroyl lysine is in the above-described ranges, the touch feeling in the coated material is satisfactory and the surface thereof is smooth, and thus the gloss is satisfactory. Further, the content of the amino acid including lauroyl lysine in the pigment composition according to the present invention is, for example, in a range of 1.0% to 10% by mass and preferably in a range of 2.0% to 8% by mass with respect to the total amount of the pigment composition.

### (Other components)

The pigment composition of the present invention may contain inorganic powder, organic powder, and the like as other components in addition to the above-described inorganic or organic pigment and the amino acid including lauroyl lysine. Examples of such inorganic powder include zirconium oxide, zinc oxide, cerium oxide, magnesium oxide, barium sulfate, calcium sulfate, magnesium sulfate, calcium carbonate, magnesium carbonate, talc, sericite, silicic acid, silicic anhydride, aluminum silicate, magnesium silicate, magnesium aluminum silicate, calcium silicate, barium silicate, strontium silicate, a metal tungstate, hydroxyapatite, vermiculite, hygilite, bentonite, montmorillonite, hectorite, zeolite, ceramic powder, dibasic calcium phosphate, alumina, aluminum hydroxide, boron nitride, and silica.

Further, examples of the organic powder include polyamide powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, nylon powder (Nylon 12 or Nylon 6), styrene acrylic acid copolymer powder, divinylbenzene styrene copolymer powder, vinyl resin powder, urea resin powder, phenol resin powder, fluorine resin powder, silicon resin powder, acrylic resin powder, melamine resin powder, epoxy resin powder, polycarbonate resin powder, microcrystalline fiber powder, and rice starch. In a case where the organic powder and the inorganic powder are used, the content thereof is, for example, in a range of 0.1% to 85% by mass and preferably in a range of 0.5% to 50% by mass with respect to the total amount of the pigment composition.

### [Method of producing pigment composition]

In a method of producing the pigment composition, for example, a wet method, a gas phase method, or a solid phase method can be used as a method of coating the surface of the inorganic or organic pigment with lauroyl lysine. The wet method is a method of chemically adsorbing lauroyl lysine on the surface of the inorganic or organic pigment in a solvent containing the inorganic or organic pigment and the lauroyl lysine, the gas phase method is a method of coating the surface of the inorganic or organic pigment with lauroyl lysine using sublimation of the lauroyl lysine, and the solid phase method is a method of physically fixing lauroyl lysine to the surface of the inorganic or organic pigment using a dry compounding device or the like. Among these, from the viewpoint of obtaining a more satisfactory pigment composition, a solid phase method is preferable in the method of producing the pigment composition according to the present invention. Further, an adsorbent may be used in the production method.

The method of coating the surface using the solid phase method is, for example, a method of premixing the inorganic or organic pigment with lauroyl lysine so that the mixture is uniformly present, stirring the inorganic or organic pigment and the lauroyl lysine using a dry particle compounding device or the like to apply a compression, a shear, and an impact thereto, and stretching the lauroyl lysine on the surface of the inorganic or organic pigment to fix the lauroyl lysine to the surface.

As the dry particle compounding device, "NOBILTA (registered trademark) NOB" (product name, manufactured by Hosokawa Micron Corporation) can be preferably used. The stirring peripheral speed in the dry particle compounding device is, for example, in a range of 5 to 20 m/sec and preferably in a range of 10 to 20 m/sec (for example, in a range of 1000 to 5000 rpm and preferably in a range of 2000 to 4000 rpm). The stirring treatment time is, for example, in a range of 1 to 10 minutes and preferably in a range of 2 to 5 minutes. The amount of lauroyl lysine to be blended is, for example, about 0.5 to 10 parts by mass with respect to 100 parts by mass of the inorganic or organic pigment.

### [Cosmetic material, ink, coating material, toner, or molded article]

The cosmetic material, the ink, the coating material, the toner, or the molded article of the present invention is not particularly limited as long as the cosmetic material, the ink, the coating material, the toner, or the molded article contains the pigment composition of the present invention. As components other than the pigment composition of the present invention, typical components suitable for the applications or purposes thereof can be blended as appropriate.

Examples of the types of the cosmetic materials include foundation (such as face color and concealers), makeup foundation (such as makeup base and pre-makeup), powder (such as face powder), lipsticks (such as lipsticks, lip rouge, lip color, lip pencils, paste rouge, lip gloss, and lip liners), eye makeup (such as eyeshadow, eye color, eyeliner, eyebrow ink, eyebrow pencils, eyebrow blush, mascara, and eyelash cosmetics), cheek cosmetics (such as blusher, cheek color, and cheek rouge), nail cosmetics (such as nail enamel, manicure, nail color, nail polish, pedicure, nail lacquer, top coat, and base coat), and hair-coloring agents (hair dyeing agents, hair color sprays, hair color sticks, color rinse, and hair manicure).

It is preferable that the cosmetic material contain, in addition the pigment composition of the present invention, an oily base for the purpose of stabilizing the dispersion of particles. Examples of such an oily base include waxes in a solid state at room temperature (25°C) and liquid oily components in a liquid state at room temperature.

Examples of the waxes include vegetable-based waxes such as candelilla wax, carnauba wax, rice wax, wood wax, and sunflower wax, animal-based waxes such as beeswax, mineral-based waxes such as ozokerite, ceresin, and microcrystalline wax, petroleum-based waxes such as solid paraffin, and synthetic waxes such as silicone wax and synthetic beeswax.

Examples of the liquid oily components include vegetable-based oils such as olive oil, castor oil, jojoba oil, macadamia nut oil, rosa canina fruit oil, cacao butter, and lanolin, animal-based oils such as horse oil, turtle oil, boar oil, mink oil, and shark oil, hydrocarbon-based oils such as petrolatum, liquid paraffin, isodecane, isododecane, octyldodecyl, and hydrogenated polyisobutene, ester oils such as isotridecyl isononanoate, isopropyl isostearate, neopentyl glycol dicaprate, isotridecyl isononanoate, glyceryl diisostearate, glyceryl triisostearate, diisostearyl malate, octyldodecanol, and di(phytosteryl/2-octyldodecyl) N-lauroyl-L-glutamate, silicone oils such as dimethylpolysiloxane and phenyl methylpolysiloxane, dimer acid ester, dimer diol derivatives, cholesterol fatty acid ester, phytosterol fatty acid ester, polyglycerin fatty acid ester, pentaerythritol fatty acid ester, and glyceryl tri-2-ethylhexanoate. In a case where the waxes and the liquid oily components are used, the content thereof is, for example, in a range of 0.1 to 10 parts by mass and preferably in a range of 0.5 to 5 parts by mass with respect to 100 parts by mass of the pigment composition.

Examples of the ink include, particularly as printing ink, lithographic (offset) ink, letterpress ink, intaglio (gravure) ink, stencil (screen) ink, flexo ink, UV curable ink, aqueous ink, oil-based ink, vegetable oil ink, newspaper ink, and ink jet ink.

Examples of the coating material include synthetic resin coating materials such as powder coating materials, aqueous (water-based) coating materials, epoxy resin coating materials, urethane resin coating materials, fluorine resin coating materials, polyester resin coating materials, melamine resin coating materials, and acrylic resin coating materials.

The toner is a microsized powder used in a laser printer or a copying machine and obtained by making colored particles adhere to plastic particles having electrostatic properties, and the pigment composition of the present invention can be used as colored particles.

The molded article is molded by using a resin containing the pigment composition of the present invention as a plastic colorant. The plastic colorant may be in any form of masterbatches (MB), colored pellets/colored compounds, dry color, or paste color/liquid masterbatches.

### EXAMPLES

Hereinafter, the present invention will be described in more detail based on examples and comparative examples. In the description of the present examples, "parts" denotes "parts by mass". Pigments of Examples 1 to 5 and Comparative Examples 1 to 12 were produced or prepared by the following methods. Further, the contact angles, the BET specific surface areas, MIU, and the gloss of the pigments were measured. The evaluation results obtained by using inorganic pigments are listed in Table 1, and the evaluation results obtained by using organic pigments are listed in Table 2.

### [Example 1]

28.5 parts of commercially available titanium oxide pigment particles (C47-060, manufactured by Sun Chemical) and 1.5 parts of commercially available lauroyl lysine (AMIHOPE LL, manufactured by Ajinomoto Co., Inc.) were premixed. Next, the mixture was treated at 5000 rpm for 5 minutes using a dry particle compounding device (NOBILTA (registered trademark), manufactured by Hosokawa Micron Corporation) (using a blade with a diameter of 86 mm) and taken out, thereby obtaining a coated pigment (1).

### [Example 2]

A coated pigment (2) was obtained in the same manner as in Example 1 except that the titanium oxide pigment particles were changed to iron oxide pigment particles (C33-8001, manufactured by Sun Chemical) and the rotation speed of the dry particle compounding device was changed to 5500 rpm in Example 1.

### [Example 3]

A coated pigment (3) was obtained in the same manner as in Example 1 except that the titanium oxide pigment particles were changed to Blue No. 1 (C39-7733, manufactured by Sun Chemical) in Example 1.

### [Comparative Example 1]

5.0 parts of commercially available titanium oxide pigment particles (C47-060, manufactured by Sun Chemical), 0.36 parts of commercially available lauroyl lysine (AMIHOPE (registered trademark) LL, manufactured by Ajinomoto Co., Inc.), 2.5 parts of calcium chloride, 118.75 parts of ethanol, and 6.25 parts of water were blended in these proportions, and the mixture was shaken with a paint conditioner for 2 hours. After the mixture was shaken, the mixture was filtered and washed with a sufficient amount of water to remove calcium chloride, thereby obtaining a wet cake of a pigment composition formed by coating the titanium oxide particles with lauryl lysine. Thereafter, the wet cake was dried and pulverized, thereby obtaining a coated pigment (4).

### [Comparative Example 2]

A blended pigment (5) was obtained by blending 28.5 parts of commercially available titanium oxide pigment particles (C47-060, manufactured by Sun Chemical) and 1.5 parts of commercially available lauroyl lysine (AMIHOPE (registered trademark) LL, manufactured by Ajinomoto Co., Inc.) .

### [Comparative Example 3]

The commercially available titanium oxide pigment particles (C47-060, manufactured by Sun Chemical) were evaluated.

### [Comparative Example 4]

A blended pigment (6) was obtained in the same manner as in Comparative Example 2 except that the titanium oxide pigment particles were changed to iron oxide pigment particles (C33-8001, manufactured by Sun Chemical) in Comparative Example 2.

### [Comparative Example 5]

The commercially available iron oxide pigment particles (C33-8001, manufactured by Sun Chemical) were evaluated.

### [Comparative Example 6]

A blended pigment (7) was obtained in the same manner as in Comparative Example 2 except that the titanium oxide pigment particles were changed to Blue No. 1 (C39-7733, manufactured by Sun Chemical) in Comparative Example 2.

### [Comparative Example 7]

The commercially available Blue No. 1 (C39-7733, manufactured by Sun Chemical) was evaluated.

### [Comparative Example 8]

The procedures were the same as in Comparative Example 1 except that the titanium oxide pigment particles were changed to Blue No. 1 (C39-7733, manufactured by Sun Chemical) in Comparative Example 1, but Blue No. 1 was dissolved, and thus a coated pigment could not be obtained.

### [Comparative Example 9]

28.5 parts of commercially available titanium oxide pigment particles (C47-060, manufactured by Sun Chemical) and 1.5 parts of commercially available N-acyl amino acid (N-lauroyl sarcosine, manufactured by Tokyo Chemical Industry Co., Ltd.) were premixed. Next, the mixture was treated at 5000 rpm for 5 minutes using a dry particle compounding device (NOBILTA, manufactured by Hosokawa Micron Corporation) (using a blade with a diameter of 86 mm) and taken out, thereby obtaining a coated pigment (8).

### [Comparative Example 10]

A coated pigment (9) was obtained in the same manner as in Comparative Example 1 except that the titanium oxide pigment particles were changed to iron oxide pigment particles (C33-8001, manufactured by Sun Chemical) in Comparative Example 1.

### [Example 4]

A coated pigment (10) was obtained in the same manner as in Example 3 except that Blue No. 1 was changed to Red NO. 202 (C19-7703, manufactured by Sun Chemical) in Example 3.

### [Comparative Example 11]

The commercially available Red No. 202 (C19-7703, manufactured by Sun Chemical) was evaluated.

### [Example 5]

A coated pigment (11) was obtained in the same manner as in Example 3 except that Blue No. 1 was changed to Yellow No. 4 (C69-7724, manufactured by Sun Chemical) in Example 3.

### [Comparative Example 12]

The commercially available Yellow No. 4 (C69-7724, manufactured by Sun Chemical) was evaluated.

### [Measurement of contact angle (evaluation of hydrophobicity)]

A pressure of about 30 MPa was applied to the pigment powder sample so that the sample was compacted, and the hydrophobicity was evaluated by measuring the contact angle of the obtained tablet with respect to water. 1.0 µL of water was added dropwise to the pigment tablet using a contact angle meter (DMo-602, manufactured by Kyowa Interface Science Co., Ltd.), and the contact angle 1.0 seconds after the dropwise addition was measured.

### [BET specific surface area]

The BET specific surface area was measured by a nitrogen adsorption method. The measurement was performed using a fully automatic specific surface area measuring device Macsorb HM model-1208 (manufactured by MOUNTECH Co., Ltd.) in conformity with "method of measuring gas adsorption amount using one-point method" specified in Appendix 2 of Japanese Industrial Standards JIS Z 8830-1990. Further, the BET specific surface area value was rounded to the second decimal place.

### [Measurement of MIU]

A sensor was placed on the sample that had been spread on a sample stand and allowed to slide to measure the friction coefficient MIU value using a friction feeling tester (KES-SE, manufactured by Kato Tech Co., Ltd.). Further, initial data with a length of 5 mm and final data with a length of 5 mm were cut, and data with a distance of 20 mm therebetween was employed. The touching feeling is smooth as the MIU value decreases.

### [Measurement of gloss]

4.0 parts of the pigment composition, 26.0 parts of NC gravure varnish (manufactured by DIC Corporation), 4.0 parts of ethyl acetate, 4.0 parts of toluene, and 2.0 parts of isopropyl alcohol were blended in these proportions, and 150 parts of 1/8 inch steel beads were added to the mixture and dispersed by a paint conditioner to prepare an ink composition. The obtained ink composition was subjected to color development with a bar coater No. 6 to form a transparent film, thereby obtaining a color-developed film. The color-developed film obtained from the ink was measured using a gloss meter (MULTI GLOSS 268, manufactured by Konica Minolta, Inc.), and the gloss value was confirmed. The gloss is satisfactory as the gloss value increases.

**[Table 1]**

| Pigment | | Pigment composition | Contact angle | BET (nitrogen) | BET(nitrogen) untreated ratio | MIU | Gloss |
|---|---|---|---|---|---|---|---|
| Titanium oxide pigment particles | Example 1 | Coated pigment (1) | 132° | 4.8 | 0.45 | 0.7 | 2.2 |
| | Comparative Example 9 | Coated pigment (8) | 10° | 5.5 | 0.52 | 1.1 | 1.4 |
| | Comparative Example 1 | Coated pigment (4) | 94° | 5.6 | 0.53 | 0.8 | 1.5 |
| | Comparative Example 2 | Blended pigment (5) | 4° | 6.1 | 0.58 | 0.7 | 1.0 |
| | Comparative Example 3 | Titanium oxide pigment particles | 0° | 10.6 | 1.00 | 1.0 | 1.0 |
| Iron oxide pigment particles | Example 2 | Coated pigment (2) | 132° | 6.1 | 0.67 | 0.9 | 1.3 |
| | Comparative Example 4 | Blended pigment (6) | 45° | 8.9 | 0.98 | 0.8 | 1.0 |
| | Comparative Example 5 | Iron oxide pigment particles | 0° | 9.1 | 1.00 | 1.0 | 1.0 |
| | Comparative Example 10 | Coated pigment (9) | 123° | 8.1 | 0.89 | 0.9 | 0.9 |

**[Table 2]**

| Pigment | | Pigment composition | Contact angle | BET (nitrogen) | BET(nitrogen) untreated ratio | MIU | Gloss |
|---|---|---|---|---|---|---|---|
| Blue No. 1 | Example 3 | Coated pigment (3) | 116° | 30.4 | 0.84 | 0.90 | 2.6 |
| | Comparative Example 6 | Blended pigment (7) | 65° | 33.4 | 0.92 | 0.90 | 1.0 |
| | Comparative Example 7 | Blue No. 1 | 59° | 36.2 | 1.00 | 1.00 | 1.0 |
| Red No. 202 | Example 4 | Coated pigment (10) | 101° | 42.0 | 0.89 | 0.8 | 1.2 |
| | Comparative Example 11 | Red No. 202 | 82° | 47.2 | 1.00 | 1.0 | 1.0 |
| Yellow No. 4 | Example 5 | Coated pigment (11) | 117° | 23.9 | 0.87 | 1.0 | 1.8 |
| | Comparative Example 12 | Yellow No. 4 | 29° | 27.4 | 1.00 | 1.0 | 1.0 |

In Tables 1 and 2, the MIU value and the gloss value are shown as relative values when the value of a base material (untreated pigment) is set to 1 because the numerical values thereof vary depending on the type of the pigment of base material particles.

In Examples 1 and 2 listed in Table 1, in which the contact angle was 95 degrees or greater and the nitrogen specific surface area ratio between before and after coating was 0.70 or less, it was found that the performance of both the MIU values and the gloss was excellent as compared with the comparative examples. In Comparative Example 9, the gloss value was 1.4, which was relatively high, but the MIU value was 1.1, the smoothness was insufficient. It was considered that the effects were not sufficiently exhibited because the degree of improvement of the smoothness was significantly smaller than those of the examples even though the smoothness was slightly improved by coating the pigment surface with the amino acid, and the hydrophobicity was also insufficient.

In Examples 3 to 5 listed in Table 2, in which the contact angle was 95 degrees or greater and the nitrogen specific surface area ratio between before and after coating was 0.90 or less, it was found that the performance of both the MIU value and the gloss was excellent as compared with the comparative examples.

Therefore, the pigment composition of the present invention exhibits the effect that the touching feeling in the coated material is satisfactory and the surface thereof is smooth, and thus the gloss is also excellent as described above.

## Claims

1. A pigment composition which is an inorganic pigment coated with lauroyl lysine,
wherein a contact angle with respect to water is 95 degrees or greater, and
a nitrogen specific surface area ratio between before and after coating is 0.70 or less.

2. A pigment composition which is an organic pigment coated with lauroyl lysine,
wherein a contact angle with respect to water is 95 degrees or greater, and
a nitrogen specific surface area ratio between before and after coating is 0.90 or less.

3. The pigment composition according to Claim 1,
wherein the inorganic pigment is a metal and/or a metal oxide.

4. The pigment composition according to Claim 3,
wherein the metal oxide is at least one or more selected from the group consisting of titanium oxide and iron oxide.

5. The pigment composition according to Claim 2,
wherein the organic pigment is at least one or more selected from Blue No. 1, Red No. 202, and Yellow No. 4.

6. A cosmetic material, an ink, a coating material, a toner, or a molded article, which contains the pigment composition according to any one of Claims 1 to 5.
